Europäisches Patentamt

European Patent Office

Office européen des brevets

(11) Publication number: **0 433 084 A2**

## (12) EUROPEAN PATENT APPLICATION

(21) Application number: **90313666.1**

(22) Date of filing: **14.12.90**

(51) Int. Cl.⁵: **A61K 31/00, A61K 31/565, A61K 31/44**

The title of the invention has been amended (Guidelines for Examination in the EPO, A-III, 7.3).

(30) Priority: **15.12.89 US 451072**
**14.11.90 US 610439**

(43) Date of publication of application:
**19.06.91 Bulletin 91/25**

(84) Designated Contracting States:
**AT BE CH DE DK ES FR GB IT LI LU NL SE**

(71) Applicant: MERCK & CO. INC.
126, East Lincoln Avenue P.O. Box 2000
Rahway New Jersey 07065-0900(US)

(72) Inventor: **Elbrecht, Alex**
**65 Maple Street**
**Watchung, NJ 07060(US)**
Inventor: **Smith, Roy G.**
**528 Forest Avenue**
**Westfield, NJ 07090(US)**

(74) Representative: **Hesketh, Alan, Dr. et al**
**European Patent Department Merck & Co.,**
**Inc. Terlings Park Eastwick Road**
**Harlow Essex, CM20 2QR(GB)**

(54) Use of aromatese inhibitors for the reversal of female sexual phenotype in poultry.

(57) Fertilized poultry embryos are treated with steroid biosynthesis inhibitors or antagonists which prevents the conversion of androgens to estrogens. By blocking the production of estrogens the genotypic female is converted into a phenotypic male. The phenotypic conversion of females to males gives the treated birds the advantage of male growth characteristics. A single administration of a steroid biosynthesis inhibitor or antagonist prior to about day 9 of embryonic incubation results in an irreversible change the sexual phenotype.

FIGURE

pUGD0603  pOV230

female
male
1976
1977
1978
1981

EP 0 433 084 A2

## REVERSAL OF FEMALE SEXUAL PHENOTYPE IN POULTRY

### BRIEF DESCRIPTION OF THE DRAWING

Figure. Identification of the W chromosome of genetic females by hybridization with plasmid pUGD0603.

### BACKGROUND OF THE INVENTION

Males of commercially important poultry species gain weight faster during the first eight weeks after hatching than do females of the same species, Nixey, 7th Eur. Poultry Conf., Vol. 2, Larbier, ed., pg. 671-679(1988). This weight gain advantage allows male birds to reach a marketable weight before females. A uniform weight gain of both male and female birds would have a significantly positive economic impact on broiler production for all important species.

Sex reversal in female chickens has been accomplished by early grafting of female chicken embryos with embryonic testes, Stoll, et al., Gen. Comp. Endocrinol. 41:66-75 (1980). The grafted testes acted during the normal period of gonadal differentiation, between 6 and 9 days of incubation, and the changes were permanent as evidenced by male characteristics in the adult birds. The sex reversed birds possess two testes associated with normally differentiated male excretory ducts and their Mullerian ducts have regressed. The development of male sex characteristics, in female genotypes, such as external features, behavior and complete spermatogenesis is evidence that these cocks have endocrine and exocrine capabilities similar to those of genotypic males.

The conversion of female genotypic embryos to male phenotype following embryonic testis grafting appears to depend on a substance or substances secreted into the blood of the female host by the grafted testis, Rashedi et al., Biol. Reprod. 29: 1221-1227 (1983). The authors propose that the masculinizing property of the grafted testes depends on anti-mullerian hormone, Mullerian-inhibiting substance (MIS), which is responsible for the regression of the Mullerian ducts during normal differentiation.

Sexual differentiation of birds is dependent upon complex physiological control of an anatomically complicated urogenital system. The female bird has an ovary and a persistent Mullerian duct on the left side and a small ovotestis and no Mullerian duct on the right compared to the male, which has bilateral testes and no Mullerian ducts. It has been observed that chick gonads, female as well as male, produce MIS which may cause regression of the right Mullerian duct in the female, Huston et al., J. Pediatr Surg. 16, 822-827 (1981). This did not answer the question of why the left duct in the female does not regress despite ovarian MIS production. It has been postulated that estrogen may protect the left duct, since exogenous estrogens cause feminization of the male chick embryo, with regression of the Mullerian ducts, Rahil and Narbaitz Gen. Comp. Endocrinol. 18: 315-318 (1972). Studies with antiestrogens, such as tamoxifen and LY117018, had little effect on the female Mullerian ducts unless given in high doses or with added testosterone, Huston et al., Gen. Comp. Endocrinol. 57: 88-102 (1985). Huston et al. also observed that a postulated aromatase inhibitor, norethindrone, caused partial regression of the upper end of the left Mullerian duct as well as complete loss of the lower ends of both ducts in the female. The authors suggested that the steroid environment is a critical factor in the response of the Mullerian ducts to MIS and that estrogen blockage may allow endogeneous MIS in the ovary to induce partial regression of the Mullerian ducts in the female chick embryo. None of the references relating to sexual differentiation of birds describe complete phenotypic transformation from females to males.

Studies using mammalian systems suggests that MIS directly modulates aromatase activity to affect gonadal differentiation. Mammalian MIS, produced only in the testes, induces the regression of the Mullerian ducts of the male fetus. Exposure of ovine fetal ovaries to MIS induced an endocrine sex reversal resulting in the release of testosterone instead of estradiol due to the suppression of aromatase activity, Vigier et al., Proc. Natl. Acad. Sci. USA 86: 3684-3688 (1989). The avian and mammalian responses to MIS and its role in differentiation are noticeably different and data gained from mammalian experiments should not be extrapolated to birds, Hutson et al., Reproduct. Physiol. IV, In Inter. Rev. Physiol. 27: 177-223 (1983).

### OBJECTS OF THE INVENTION

It is, accordingly, an objective of the present invention to provide a means of converting genotypic female birds into phenotypic male birds. Another object is to treat bird embryos with steroid biosynthesis inhibitors or antagonists to prevent the conversion of testosterone to estradiol. A further object is to treat bird embryos with an aromatase inhibitor to convert female genotypic birds into male phenotypic birds. Another object is to convert genotypic female birds into phenotypic male birds so that following hatching all birds of a flock will gain weight at the rate of males or that the converted males will gain weight at a rate greater than non-treated females. A further object is to obtain phenotypically male birds with a female genotype. Another object is to be able to irreversible change the sexual phenotype birds by treating a bird embryo with steroid biosynthesis inhibitors or antagonists prior to about day 9 of embryonic development.

## SUMMARY OF THE INVENTION

Fertilized poultry embryos are treated with steroid biosynthesis inhibitors or antagonists which prevents the conversion of testosterone to estradiol. By blocking the production of estradiol the genotypic female is converted into a phenotypic male. The phenotypic transformation of females to males gives the treated birds the advantage of male growth characteristics. A single administration of a steroid biosynthesis inhibitor or antagonist prior to about day 9 of embryonic incubation results in an irreversible change the sexual phenotype.

## DETAILED DESCRIPTION OF THE INVENTION

The present invention relates to the conversion of a genotypic female fowl to a phenotypic male fowl. Fowl is defined herein as wild or domesticated gallinaceous birds that serve as a source of meat and that include, but are not limited to, commercially important birds such as chickens, turkeys, ducks, geese, guinea fowl, pheasants, pigeons, peafowl and quail. Fowl as used herein will also include Poultry, all domesticated birds kept for meat.

Fertilized embryos of any domesticated gallinaceous bird, with chicken, goose, duck and turkey being preferred, are treated with an aromatase inhibitor to prevent the conversion of testosterone to estradiol. Aromatase is an enzyme complex incorporating a NADPH-cytochrome c-reductase and a cytochrome $P_{450}$ component which mediates the conversion of androgens to estrogens, Bellino, J. Steroid Biochem. 17: 261-270 (1982). The reaction is believed to involve three hydroxylation steps, two at the C-19 position (Meyer, Biochem. Biophys. Acta 17: 441-442 [1955]; Morato et al., Biochem. Biophys. Res. Comm. 6: 334-338 [1961]) and one at C-2 (Hahn and Fishman, J. Biol. Chem. 259: 1689-1694 [1984]; Brodie et al., J. Am. Chem. Soc. 91: 1241-1242 [1969]) which result in the conversion of the A ring of the androgen molecule to an aromatic ring. Since aromatization is a unique reaction in the biosynthesis of steroids, specific inhibitors should not cause deprivation of other essential steroids. The inhibition or blocking of the conversion of androgens (testosterone, androstenedione) to estrogens (estradiol, estrone) results in an accumulation of androgens (testosterone, androstenedione) which may allow the gonad in genetic females to differentiate into a testis and prevents the regression of the right gonad resulting in the production of phenotypic male birds from genotypic female birds. An aromatase inhibitor as defined herein is any steroidal or non-steroidal compound which prevents the conversion of androgens to estrogens. The compounds include substrate analogues of androstenedione, testosterone or other steroidal substances involved in the aromatase pathway, Henderson, J. Steroid Biochem. 27: 905-914 (1987). Non-steroidal compounds that block aromatase activity are also included within the scope of the invention. These non-steroidal compounds include compounds or analogues that can bind to the enzymatic active site of aromatase and inhibit enzymatic activity. Non-steroidal or steroidal compounds will also include compounds or analogues that bind to the enzyme at a site away from the enzymatic site and cause a structural change in the enzyme which results in a loss of enzymatic activity. Aromatase inhibitors further include non-steroidal compounds that interfere with cytochrome $P_{450}$ mediated hydroxylations such as those described by Brodie et al., J. Steroid Biochem. 27: 899-903 (1987).

The following compounds are known aromatase inhibitors as disclosed by the associated reference. The reference will also directly contain a method for making the compound or will direct one who wishes to use the compound to a method for producing the compound. The aromatase inhibitors of the instant invention include, but are not limited to, the following compounds: 6-[(1H-imidazol-1-yl)phenylmethyl]-1 methyl-1H-benzotriazole, 6-[(4-chlorophenyl)(1H-1,2,4-triazol-1-yl)methyl]-1-methyl-1H-benzotriazole as described in Publication No. 293,978; 2,2-[5-(1H-1,2,4,-triazol-1-ylmethyl)-1,3-phenylene]di(2-methylpropiononitrile), 2,2-[5-(imidazol-1-ylmethyl)-1,3-phenylene]-di(2-methylpropiononitrile), 2-[3-(1-hydroxy-1-methylethyl)-5-(1H-1,2,4-triazol-1-ylmethylphenyl]-2-methylpropiononitrile, 2,2-[5-dideuterio(1H-1,2,4-triazol-1-yl)methyl-1,3-phenylene]di(2trideuteriomethyl-3,3,3-(trideuteriopropiononitrile), and 2,2-[5-dideuterio(1H-1,2,4-triazol-1-ylmethyl-1,3-phenylene)di(2-methylpropiononitrile) as disclosed in European Patent Application, Publication No. 296,749; 1,1-dimethyl-8-(1H-1,2,4-triazol-1-ylmethyl)-2(1H)-naphtho[2,1-b] furanone, 1,2-dihydro-1,1-dimethyl-2-oxo-8-(1H-1,2,4-triazol-1-ylmethyl)naphtho-[2,1-b]furan-7-carbonitrile, 1,2-dihydro-1,1-dimethyl-2-oxo-8-(1H-1,2,4-triazol-1-ylmethyl)-naphtho[2,1-b]furan-7-carboxamide, and 1,2-dihydro-1,1-dimethyl-2-oxo-8-[di(1H-1,2,4-triazol-1 yl)methyl]naphtho[2,1-b]furan-7-carbonitrile as disclosed in European Patnet Application, Publication No. 316,097; 2-(4-chlorobenzyl)-2-fluoro-1,3-di(1,2,4-triazol-1-yl)-propane, 2-fluoro-2-(2-fluoro-4-chlorobenzyl)-1,3-di(1,2,4-triazol-1-yl)propane, 2-fluoro-2-(2-fluoro-4-trifluoromethylbenzyl)-1,3-di(1,2,4-triazol-1-yl)propane, 3-(4-chlorophenyl)-1-(1,2,4-triazol-1-yl)-2-(1,2,4-triazol-1-ylmethyl)butan-2-ol, 2-(4-chloro-α-fluorobenzyl)-1,3-di(1,2,4-triazol-1-yl)propan-2-ol, 2-(4-chlorobenzyl)-1,3-bis(1,2,4-triazol-1-yl)propane, 4-[2-(4-chlorophenyl)-1,3-di(1,2,4-triazol-1-ylmethyl)ethoxymethyl]-benzonitrile,1-(4-fluorobenzyl)-2-(2-fluoro-4-trifluoromethylphenyl)-1,3-di(1,2,4-triazol-1-yl)propan-2-ol, 2-(4-chlorophenyl)-1-(4-fluorophenoxy)-1,3-di(1,2,4-triazol-1-yl)propan-2-ol, 1-(4-cyanobenzyl)-2-(2,4-difluorophenyl)-1,3-di(1,2,4-triazol-1-yl)-propan-2-ol and 2-(4-chlorophenyl)-1-phenyl-1,3-di(1,2,4-triazol-1-yl)-propan-2-ol as described in European Patent Application, Publication No. 299,684; 5-bis(4-chlorophenyl)-methylpyrimidine as disclosed in U.S. Patent No. 4,762,836; α,α-bis(4-chlorophenyl)-2-pyrazinemethanol as described in U.S. Patent No. 4,764,376; N-(2,4-difluorophenyl)-N-benzyl-3-pyridinemethanamine and N-(2-chlorophenyl-α-(4-fluorophenyl)-3-pyridinemethanamine as disclosed in U.S. Patent No. 4,744,251; 1-(10,11-dihydro-5H-dibenzo[a,d]cyclohepten-5-yl)-1H-imidazole and 1-(9H-fluoren-9-yl)-1H-imidazole as disclosed in U.S. Patent No. 4,757,082; 3-bis(4-chlorophenyl)-3-methylpyridine and α,α-bis(4-chlorophenyl)-3-pyridinemethanol as disclosed in U.S. Patnet No. 4,757,076; 5H-5-(4-cyanophenyl)-6,7-dihydropyrrolo[1,2-c]-imidazole and 5H-5-(4-cyanophenyl)-6,7,8,9-tetrahydroimidazo[1,5-a]azepine and disclosed in U.S. Patent No. 4,728,645; 5-[(1 H-imidazol-1-yl)phenylmethyl]-2-methyl-1 H-benzimidazole and 5-[(3-chlorophenyl)(1 H-imidazol-1-yl)-methyl]-1 H-benzimidazole as disclosed in European Patent Application, Publication No. 260,744; (Z)-α-(1,2,4-triazol-1-ylmethyl)stilbene-4,4'-dicarbonitrile, (Z)-4'-chloro-α-(1,2,4-triazol-1-ylmethyl)-stilbene-4-carbonitrile, (Z)-α-(1,2,4-triazol-1-ylmethyl)-4'-(trifluoromethyl)stilbene-4-carbonitrile, (E)-ß-fluoro-α-(1,2,4-triazol- 1-ylmethyl)stilbene-4,4'-dicarbonitrile, (Z)-4'-fluoro-α-(imidazol-1-ylmethyl)stilbene-4-carbonitrile, (Z)-2',4'-dichloro-α-(imidazol-1-ylmethyl)stilbene-4-carbonitrile, (Z)-4'-chloro-α-(imidazol-1-ylmethyl)stilbene-4-carbonitrile, (Z)-α-(imidazol-1-ylmethyl)stilbene4,4'-dicarbonitrile,(Z)-α-(5-methylimidazol-1-ylmethyl)stilbene-4,4'-dicarbonitrile, and (Z)-2-[2-(4-cyanophenyl)-3-(1,2,4-triazol-1-yl)propenyl]pyridine-5-carbonitrile as disclosed in European Patnet Application, Publication No. 299,683; (1R* ,2R*)-6-fluoro-2-(4-fluorophenyl)-1,2,3,4-tetrahydro-1-(1H-1,2,4-triazol-1-ylmethyl)naphthalene, (1R* ,2R*)-6-fluoro-2-(4-fluorophenyl)-1,2,3,4-tetrahydro-1-(1H-imidazolylmethyl)-naphthalene, (1R* ,2R*)-and (1R* ,2S*)-2-(4-fluorophenyl)-1,2,3,4-tetrahydro-1-(1H-1,2,4-triazol-1-ylmethyl)naphthalene-6-carbonitrile, (1R* ,2R*)-and (1R* ,2S*)-2-(4-fluorophenyl)-1,2,3,4-thetahydro-1-(1H-imidazolylmethyl)naphthalene-6-carbonitrile, (1R* ,2R*)-and (1R* ,2S*)-1,2,3,4-tetrahydro-1-(1H-1,2,4-triazol-1-ylmethyl)naphthalene-2,6-dicarbonitrile, (1R* ,2R*)-and (1R* ,2S*)-1,2,3,4-tetrahydro-1-(1H-imidazol-1-ylmethyl)naphthalene-2,6-dicarbonitrile, and(1R* ,2S*)-2-(4-fluorophenyl)-1,2,3,4-tetrahydro-1-(5-methyl-1H-imidazolylmethyl)naphthalene-6-carbonitrile as disclosed in European Patent Application, Publication No. 281,283; 8-chloro-5-(4-chlorophenyl)-5H-indeno[1,2-d]pyrimidine as disclosed in U.S. Patnet No. 4,769,378; 5-bis (4-chlorophenyl) methylpyrimidine as disclosed in U.S. Patent No. 4,762,836; 10-(2-propynyl)-estr-4-ene-3,17-dione as disclosed in U.S. Patent No. 4,322,416; 6-[(4-chlorophenyl) (1H-1,2,4-triazol-1-yl) methyl]-1-methyl-1H-benzotriazole as described in European Patent Application, Publication No. 293,978; 1-methylandrosta-1,4-dien-3,17-dione as disclosed in U.S. Patent No.4,591,585; 3-ethyl-3-(4-pyridyl)piperidine-2,6-dione as disclosed in British Patent GB 2,151,226; 4-hydroxyandrostene-3,17-dione as disclosed in U.S. Patent No. 4,500,523; 4-(5,6,7,8-tetrahydroimidazo[1,5-a]pyridin-5yl)benzonitrile as disclosed in U.S. Patent No. 4,728,645; 6-methyleneandrosta-1,4-diene-3,17-dione, 4-aminoadrostan-1,4,6-trien-3,17-dione and 4-aminoandrosta-4,6-diene-3,17-dione as disclosed in U.S. Patent No. 4,757,061; 3-(1H-imidazol-1-ylmethyl)-2-methyl-1H-indole-1-propanoic acid as disclosed in U.S. Patent No. 4,273,782; 5-[3-chlorophenyl)(1H-imidazol-1-yl)methyl]-1H-benzimidazole as disclosed in European Patent Application, Publication No. 260,744; 10ß-thiiranylestr-4-ene-3,17-dione and 10ß-oxiranylestr-4-ene-3,17-dione as disclosed in J. Organ. Chem. 53: 5947-5951 (1988); 3-ethyl-3-(4-pyridyl)piperidine-2,6-dione as described in U.S. Patent No. 4,668,689; 3-(4-aminophenyl)-3-ethyl-pyrrolidine-2,5-dione as disclosed in J. Med. Chem. 29: 520-523 (1986); 1-(7-carboxyheptyl)-imidazole as described in U.S. Patent No. 4,320,134; 1,1-dimethyl-8-(1H-1,2,4-triazol-1-ylmethyl)-2 (1H)-naphtho [2,1-b]furanone (1a) as disclosed in European Patent Application, Publication No. 316,097; ± 5-(p-cyanophenyl)-5, 6, 7, 8-tetrahydroimidazo [1, 5-a] pyridine hydrochloride as disclosed in U.S. Patent No. 4,617,307; 1,4,6-androstatriene-3,17-dione disclosed in Biochem. Pharmac. 31: 2017-2023 (1982) and other compounds well known in the art of aromatase inhibition and cancer therapy such as bis-(p-cyanophenyl)-imidazo-1-yl-methane hemisuccinate and pharmaceutically acceptable derivatives, acid addition salts and possible stereochemically isomeric forms thereof, if and where appropriate. The invention is also intended to include any biologically active equivalents of an aromatase inhibitor as described above.

4

Fertilized poultry embryos, preferably chicken turkey, duck or goose, are treated on about day 1 to about day 9 of incubation with one or more aromatase inhibitors. Preincubation as used herein is considered to be day 0, thus day 1 is the day the eggs are placed in the incubator. Incubation begins when the embryos are placed in an incubator. Treatment is by one or more injections of the aromatase inhibitors into the embryo or by dipping the embryonated eggs one or more times in a solution of the aromatase inhibitors. A single injection is given on about day 1 to about day 9 of incubation. Multiple injections are given every other or every third day starting at about day 1 of incubation and are continued through about day 9 of incubation. Dipping of embryos is by a single treatment or multiple treatments at times discussed above. Dipping as used herein also includes pressure impregnating fertilized eggs with one or more aromatase inhibitors. The aromatase inhibitor or inhibitors are dissolved or suspended in a physiologically acceptable carrier. Such aqueous physiologically acceptable carriers include, but are not limited to water, saline, physiologic saline, buffer saline, phosphate buffered saline, phosphate buffered saline glucose. Non-aqueous physiologically acceptable carriers include, but are not limited to, 1,2-propanediol, dimethyl sulfoxide, ethanol and the like. The concentration of the aromatase inhibitor or inhibitors is from about 0.1 mg/ml to about 100 mg/ml and the injection volume ranges from about 10 $\mu$l to about 100 $\mu$l producing doses of about 10 $\mu$g to about 10 mg. Injections are made by hand or by machine with about 23 G by 1 in needles. The injection is made under the air sac into the egg white surrounding the developing embryo. Administration of the aromatase inhibitor is also possible by injections made into the air sac without piercing the membrane. Administration is also possible by injection into the opposite end of the egg directly into the albumin. The injection hole is sealed with Scotch tape, wax, glue and the like. The aromatase inhibitor can also be administered by dipping the egg in a solution containing one or more inhibitors at concentrations as described above. Injected or dipped eggs are placed in a humidified, rocking incubator and allowed to develop and hatch.

Conversion to male phenotype is determined at about day 14 of incubation, at hatching and post-hatching. Pre-hatching conversion is determined by removing the embryos from their shells and dissecting to observe development of the gonads. Male phenotype is determined following hatching by organ sex characterization and/or by vent sexing which are known in the art. The conversion of the genotypic female poultry to the phenotypic male poultry results in secondary sex characters such as increased weight gain and/or an improved lean to fat ratio.

The inhibition of aromatase activity prior to about day 9 of embryogenesis triggers a developmental switch which causes the indifferent gonad of the genetically female embryo to differentiate into a testis rather than an ovary. The present invention is unique that a single treatment which results in the inhibition of aromatase activity results in an irreversibly change in the normal program for sexual differentiation. This time and substance treatment results in a female chicken developing a male phenotype. The irreversible nature of the reversal is illustrated by the fact that the genotypic female has all the behavioral characteristics of males. Most significant is the fact that upon attaining sexual maturity, which occurs at about seven months after a single treatment with an aromatase inhibitor, the testes of the genetic female produce sperm.

A major advantage of treating developing embryos with active aromatase inhibitors is the inhibition of the conversion of androgens to estrogens which normally takes place in developing female poultry. This inhibition results in an increase in the ratio of androgens to estrogens which induces or, converts the embryo to, the male phenotype without having to treat the embryos with androgens or other hormones.

The following examples illustrate the present invention without, however, limiting the same thereto.

EXAMPLE 1

Phenotypic Conversion Of Line 139 White Leghorn Chickens By ± 5- (p-cyanophenyl)-5, 6, 7, 8-tetrahydroimidazo [1,5-α] pyridine hydrochloride

Fertilized chicken embryos were injected on day 4 or 5 of incubation with the aromatase inhibitor ± 5-(p-cyanophenyl)-5, 6, 7, 8-tetrahydroimidazo [1, 5-α] pyridine hydrochloride. The inhibitor was dissolved in saline at concentrations ranging form 1 mg/ml to 100 mg/ml and the injection volume ranged from 20 $\mu$l to 100 $\mu$l producing doses of 20 $\mu$g to 10 mg. Injections were made with a 23 G x 1 inch needle. A light source was used to orient the eggs and the injection is made under the air sac into the egg white surrounding the developing embryo. The hole was sealed with Scotch tape and the eggs were placed in a humidified, rocking incubator. At day fourteen of incubation, the embryos were removed form their shells and dissected to observe development of the gonads. The results are shown in the following table.

## TABLE 1

### GONADAL SEX TYPE OF 14 DAY EMBRYOS
### TREATED WITH AROMATASE INHIBITOR

| Amount injected | Number injected | Survived day 14 | Male | Female | Und.[1] |
|---|---|---|---|---|---|
| **Experiment 1** | | | | | |
| 20 µg | 4 | 2 | 0 | 2 | 0 |
| 100 µg | 4 | 3 | 3 | 0 | 0 |
| 2 mg | 4 | 2 | 2 | 0 | 0 |
| 10 mg | 4 | 0 | 0 | 0 | 0 |
| **Experiment 2** | | | | | |
| Saline | 5 | 5 | 3 | 2 | 0 |
| 100 µg | 5 | 4 | 3 | 0 | 1 |
| 1 mg | 6 | 4 | 4 | 0 | 0 |
| **Experiment 3** | | | | | |
| Saline | 8 | 7 | 4 | 3 | 0 |
| 100 µg | 7 | 5 | 4 | 0 | 1 |
| 1 mg | 7 | 5 | 5 | 0 | 0 |

[1]  Undetermined

[2]  The birds in experiments 1 and 3 were injected on day 5 while those in experiment 2 were injected on day 4.

The results demonstrate that female chicken embryos treated with ± 5- (p-cyanophenyl)-5, 6, 7, 8-tetrahydroimidazo [1, 5-α] pyridine hydrochloride are converted to phenotypic male embryos. The conversion is dose dependent and is manifest by day 14 of incubation.

A large experimental group of Line 67 White Leghorn embryos, 193 fertilized eggs, was injected with 1mg of ± 5- (p-cyanophenyl)-5, 6, 7, 8-tetrahydroimidazo [1, 5-α] pyridine hydrochloride while 100 control embryos received saline, 100µl. Following hatching the birds were vent sexed. Of the 75 that hatched from the experimental group all birds vent sexed as males. The surviving control birds, 60, were evenly divided between male, 30, and female, 30. These results demonstrate that the aromatase inhibitor converts the phenotype of the female to that of the male.

Phenotypic conversion was further demonstrated in an experiment which compared the concentration of ± 5- (p-cyanophenyl)-5, 6, 7, 8-tetrahydroimidazo [1, 5-α] pyridine hydrochloride and date of injection. The procedures are the same as described above. The results are shown in the following table.

## TABLE 2
### VENT SEX TYPE OF HATCHED CHICKES
### TREATED IN OVO WITH AROMATASE INHIBITOR

| Group | Amount injected | Number injected | Day | Hatched | Male | Female |
|-------|-----------------|-----------------|-----|---------|------|--------|
| A | 1 mg | 100 | 1 | 9 | 7 | 2 |
| B | 1 mg | 100 | 3 | 2 | 2 | 0 |
| C | 1 mg | 90 | 5 | 46 | 46 | 0 |
| D | 0.5 mg | 90 | 5 | 36 | 36 | 0 |
| E | 0.1 mg | 90 | 5 | 52 | 52 | 0 |
| F | Saline | 90 | 5 | 56 | 26 | 30 |
| G | Non-Injected | 90 | - | 69 | 40 | 29 |
| H | 1 mg | 90 | 7 | 38 | 38 | 0 |

These results show that the aromatase inhibitor is active over a rather broad concentration range and can be given at various times in the early development of the embryo.

Phenotypic conversion was further demonstrated in a dose titration experiment which compared concentrations of ± 5- (p-cyanophenyl)-5, 6, 7, 8-tetrahydroimidazo [1, 5-α] pyridine hydrochloride given on the same day. The procedures are the same as described above. The results are shown in the following table.

## TABLE 3

### Dose Tritation of Abor Acre x Abor Acre Hybrid Broiler Chickens

| Day Injected | Amonunt Injected | Male | Female | %Hatched |
|---|---|---|---|---|
| 0 | 50 µg | 45 | 2 | 40 |
| 5 | 10 µg | 60 | 36 | 72 |
| 5 | 50 µg | 88 | 0 | 67 |
| 5 | 100 µg | 91 | 0 | 69 |
| 5 | Saline | 39 | 44 | 66 |
| 5 | Untreated | 56 | 52 | 81 |

These results further illustrate the dose dependency of the aromatase inhibitor.

EXAMPLE 2

Phenotypic Conversion Of Turkeys By ± 5- (p-cyanophenyl)-5, 6, 7, 8-tetrahydroimidazo [1, 5-α] pyridine hydrochloride

Fertilized turkey embryos were injected on day 4 or 5 of incubation with the aromatase inhibitor (± 5-(p-cyanophenyl)-5, 6, 7, 8-tetrahydroimidazo [1, 5-α] pyridine hydrochloride as described in Example 1. Turkeys exhibiting sex-linked feather color were used to evaluate the effect of the aromatase inhibitor to alter the phenotypic sex of turkeys. The turkeys were from the Norfolk Black strain in which the progeny of the Norfolk Black hens and auburn males sex link as black male progeny and auburn females. A second system exploited the auburn:bronze feather sexing system. Injections were made with a 23 G x 1 inch needle. A light source was used to orient the eggs and the injection is made under the air sac into the egg white surrounding the developing embryo. The hole was sealed with Scotch tape and the eggs were placed in a humidified, rocking incubator. The birds were allowed to hatch and the phenotypic and genotypic sex determined. Four birds hatched from the eggs inoculated with 1 mg aromatase inhibitor. Two feather-sexed as female and 2 as male. All 4 vent sexed as male. There were 2 pipped embryos, 1 live and 1 dead, and 2 embryos that died after 25 and 27 days of incubation. All embryos were phenotypic male.

EXAMPLE 3

Phenotypic Conversion Of F₁ Hybrids of Rhode Island Males Crossed With Plymouth Barred Rock Females By 1,4,6-androstatriene-3,17-dione

Fertilized chicken embryos were injected on day 5 of incubation with the aromatase inhibitor 1,4,6-androstatriene-3,17-dione. The inhibitor was dissolved in 1,2-propane-diol at a concentration of 20 mg/ml and the injection volume was 100 μl producing a dose of 2 mg. Injections were made with a 23 G x 1 inch needle. A light source was used to orient the eggs and the injection is made under the air sac into the egg white surrounding the developing embryo. The hole was sealed with Scotch tape and the eggs were placed in a humidified, rocking incubator. At day fourteen of incubation, the embryos were removed form their shells and dissected to observe development of the gonads. The results are shown in the following table.

## TABLE 4

## GONADAL SEX TYPE OF 14 DAY EMBRYOS
## TREATED WITH AROMATASE INHIBITOR

| Amount injected | Day of injection | Survived day 14 | Male | Female |
| --- | --- | --- | --- | --- |
| 2.0 mg | 5 | 5 | 5 | 0 |
| Vehicle | 5 | 3 | 1 | 2 |

These results show that the aromatase inhibitor was active in converting female genotypes to male phenotypes as evidenced by 100 % of the birds being males.

EXAMPLE 4

Effects of ± 5- (p-cyanophenyl)-5, 6, 7, 8-tetrahydroimidazo [1, 5-α] pyridine hydrochloride On Chicken Development

Fertilized chicken embryos were injected on day 4 or 5 of incubation with the aromatase inhibitor (± 5-(p-cyanophenyl)-5, 6, 7, 8-tetrahydroimidazo [1, 5-α] pyridine hydrochloride as described in Example 1. After hatching, treated females appeared and behaved similar to control females for approximately two months. At about this time the treated females began to develop large combs and wattles, aggressive behavior, and a tendency to crow, all characteristics of genetic males. These secondary sex differences became more apparent with time and by seven months of age the treated females were obviously masculine in appearance and behavior.

To more easily monitor changes in development as a function of age according to genetic sex two different autosexing strains of chickens were treated as above. All chickens were allowed to reach sexual maturity. Using Sex-Sal (Avian Services, Frenchtown, PA) one of two treated females developed a male phenotype. Embryos of the hybrids used in Example 3 were treated and three of four adult females developed male phenotypes. One of the three was killed after 24 weeks and the remaining two (tag numbers 1977 and 1981) were killed after 31 weeks. Prior to killing blood was taken from each animal and used to determine sex steroid levels. At the time of killing the gonads were removed and fixed for histology in Bouin's fixative.

When the abdominal cavities ot treated, 31 week old, females were opened several differences from control females were noted. The most obvious of these was the absence of abdominal fat. At this age controls were sexually mature and had been laying eggs for approximately one month. Serum estrogen levels were high (Table 5), and the turbidity from circulating lipids was also obvious. In contrast, treated females had serum hormone profiles resembling adult males (Table 5). The difference in lipid levels was also apparent in the liver. In controls, the livers were fatty and yellow in color.

## TABLE 5
### Sex Steroid Hormone Levels

|  | Estradiol (ng/ml) | Testosterone (ng/ml) |
|---|---|---|
| Control female | 129 | * |
| Control male | 2 | 219 |
| 1976 | 57 | - |
| 1977 | 12 | 224 |
| 1978 | 5 | 412 |
| 1981 | 2 | 109 |

Estradiol and testosterone levels were determined in serum samples using commercial RIA assay kits which are well known in the art. Testosterone levels of samples marked with an asterisk could not be determined accurately because of high lipid levels in the serum. The animal tagged 1976 was a genetic female that did not respond to treatment with ± 5- (p-cyanophenyl)-5, 6, 7,8-tetrahydroimidazo [1, 5-α] pyridine hydrochloride. It was phenotypically female and was laying eggs at the time of sacrifice. Animal 1978 is a treated, genetic male and displayed no obvious changes because of treatment. Animals 1977 and 1981 are genetic females but phenotypic males.

The right gonad and oviduct of the normal female begins to regress during embryogenesis and at maturity females have a single ovary and oviduct. The remaining left ovary consists of a series of follicles staged to mature at daily intervals. In contrast females treated with an aromatase inhibitor possess two gonads without oviducts. The gonads appear somewhat smaller and more irregular than the testes of a normal male of the same age, however, the gonads clearly resemble testes. The presence of testes was observed in three of the four treated females in this experiment.

Histological examination of gonads from control males and treated females at 31 weeks of age was carried out on tissue fixed in Bouin's solution prior to embedding in paraffin. Sections were stained with hematoxylin and eosin. Normal sexually active males exhibited seminiferous tubules with interstitial cells. Normal spermatogenesis is characterized by the presence of Sertoli cells, spermatogonia, primary and secondary spermatocytes, round spermatids and elongated spermatids. Males treated with the aromatase inhibitor exhibited normal spermatogenesis and the cell associations in the seminiferous epithelium could not be distinguished from control testis. Seminiferous tubules are present in both the left and right gonads of treated females. At higher magnification, the seminiferous epithelium appears normal, again with clearly evident Sertoli cells spermatogonia, spermatocytes, and round, as well as elongating spermatids. In some areas of the tubules, there was evidence of spermatocyte and spermatid nuclei which had apparently undergone division without cytoplasmic cleavage. In some areas condensation of the spermatid nuclei appeared abnormal and this was associated with the appearance of large vacuoles. Two of the gonads examined also had a small cyst-like structure present. Nevertheless, the histological appearance of the testes of the sex-reversed females was similar in size and germ cellular associations to that observed in the normal male testis. The stages within an individual tubule from female "testis" frequently exhibited more heterogeneity in the stage of spermatogenesis within an individual cross-section than in the seminiferous epithelium from the normal male.

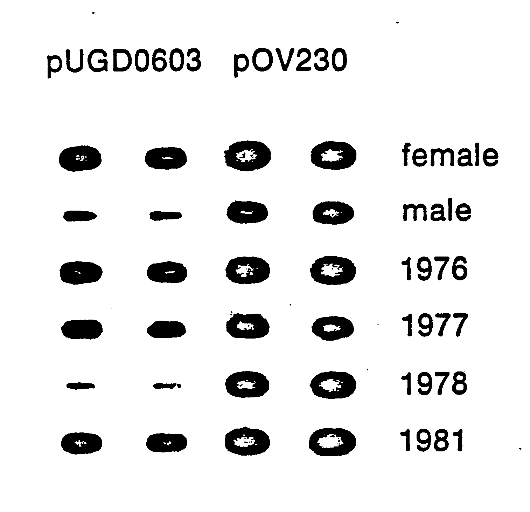

Although genetic sex can be identified by feather color in the autosexing line used for these experiments, for confirmation we also assayed for the presence of the W chromosome. Approximately 50% of the W chromosome consists of a family of repeated DNA sequences termed *Xho* 1 repeats. The plasmid pUDG0603 contains a member of that family, Kodama et al., Chromosoma 96: 18-25 (1987). The plasmid was used to probe genomic DNA isolated from chicken blood (Figure 1). Genomic DNA was isolated from chicken blood clots by digestion with proteinase K and phenol extraction. DNA ,10 $\mu$g, was loaded onto a nitrocellulose filters. The filters were prehybridized for 5 h at 42° C in a solution of 45% formamide, 4 x SSC, 0.1 M sodium phosphate, pH 6.5, 5 x Denhart's solution, 0.1% sodium pyrophosphate, 0.1% sodium dodecyl sulphate (SDS), and 0.25 mg/ml sheared denatured salmon sperm DNA. The hybridization buffer was identical except that it contained 1 x Denhart's solution, 0.1 mg/ml salmon sperm DNA and contained in addition 10% dextran sulphate. The filters were hybridized at 42° C for 16 h with nick-translated plasmid pUDG0603 or pOV230. The blots were washed to a final stringency of 0.5 x SSC, 0.5% SDS, at 68° C for 30 min and subjected to autoradiography. The same filter was used for both hybridizations.

Although there is some hybridization to DNA isolated from a control male, the signal with control female DNA is much more intense. Chickens 1976, 1977 and 1981 are clearly identified as genetic females. To ensure that comparable amounts of DNA were loaded for each sample, the blot was stripped and rehybridized with a plasmid containing cDNA sequences from the chicken ovalbumin gene, McReynolds et al., Gene 2: 217-230 (1977). The presence of the W chromosome in the phenotypic males 1977 and 1981 identified them as genetic females.

## Claims

1.  A method for converting genotypic female poultry into phenotypic male poultry comprising administering to a healthy poultry embryo a phenotypic converting amount of a substance which prevents the conversion of androgens to estrogens and results in the conversion of genotypic female poultry into phenotypic male poultry.

2.  A method for converting genotypic female poultry into phenotypic male poultry comprising administering to a healthy poultry embryo a phenotypic converting amount of a substance which prevents the conversion of testosterone or androstenedione to estradiol or estrone and results in the conversion of genotypic female poultry into phenotypic male poultry.

3.  A method for converting genotypic female poultry into phenotypic male poultry comprising administering to a healthy poultry embryo a phenotypic converting amount a an aromatase inhibitor.

4.  The aromatase inhibitors of claim 3 wherein the said aromatase inhibitor is a steroid substrate analogue or a non-steroid analogue which binds and inactivates aromatase or is a compound that inhibits the cytochrome P450 component of the aromatase complex.

5.  The aromatase inhibitor of claim 3 wherein the said aromatase inhibitor is selected from the group consisting of: 6-[(1H-imidazol-1-yl)phenylmethyl]-1 methyl-1H-benzotriazole, 6-[(4-chlorophenyl)(1H-1,2,4-triazol-1-yl)methyl]-1-methyl-1H-benzotriazole as described in Publication No. 293,978; 2,2-[5-(1H-1,2,4,-triazol-1-ylmethyl)-1,3-phenylene]di(2-methylpropiononitrile), 2,2-[5-(imidazol-1-ylmethyl)-1,3-phenylene]-di(2-methylpropiononitrile), 2-[3-(1-hydroxy-1-methylethyl)-5-(1H-1,2,4-triazol-1-ylmethylphenyl]-2-methylpropiononitrile, and 2,2-[5-dideuterio(1H-1,2,4-triazol-1-yl)methyl-1,3-phenylene]di(2trideuteriomethyl-3,3,3-(trideuteriopropionitrile), and 2,2-[5-dideuterio(1H-1,2,4-triazol-1-ylmethyl-1,3-phenylene)di(2-methylpropiononitrile); 1,1-dimethyl-8-(1H-1,2,4-triazol-1-ylmethyl)-2(1H)-naphtho[2,1-b] furanone, 1,2-dihydro-1,1-dimethyl-2-oxo-8-(1H-1,2,4-triazol-1-ylmethyl)naphtho-[2,1-b]-furan-7-carbonitrile, 1,2-dihydro-1,1-dimethyl-2-oxo-8-(1H-1,2,4-triazol-1-ylmethyl)-naphtho[2,1-b]furan-7-carboxamide, and 1,2-dihydro-1,1-dimethyl-2-oxo-8-[di(1H-1,2,4-triazol-1 yl)methyl]naphtho[2,1-b]furan-7-carbonitrile; 2-(4-chlorobenzyl)-2-fluoro-1,3-di(1,2,4-triazol-1-yl)propane, 2-fluoro-2-(2-fluoro-4-chlorobenzyl)-1,3-di(1,2,4-triazol-1-yl)propane, 2-fluoro-2-(2-fluoro-4-trifluoromethylbenzyl)-1,3-di(1,2,4-triazol-1-yl)propane, 3-(4-chlorophenyl)-1-(1,2,4-triazol-1-yl)-2-(1,2,4-triazol-1-ylmethyl)butan-2-ol, 2-(4-chloro-$\alpha$-fluorobenzyl)-1,3-di(1,2,4-triazol-1-yl)propan-2-ol, 2-(4-chlorobenzyl)-1,3-bis(1,2,4-triazol-1-yl)-propane, 4-[2-(4-chlorophenyl)-1,3-di(1,2,4-triazol-1-ylmethyl)ethoxymethyl]-benzonitrile,1-(4-fluorobenzyl)-2-(2-fluoro-4-trifluoromethylphenyl)-1,3-di(1,2,4-triazol-1-yl)propan-2-ol, 2-(4-chlorophenyl)-1-(4-fluorophenoxy)-1,3-di(1,2,4-triazol-1-yl)propan-2-ol, 1-(4-cyanobenzyl)-2-(2,4-difluorophenyl)-1,3-di(1,2,4-triazol-1-yl)-propan-2-ol and 2-(4-chlorophenyl)-1-phenyl-1,3-di(1,2,4-triazol-1-yl)-propan-2-ol; 5-bis(4-chlorophenyl)methylpyrimidine; $\alpha,\alpha$-bis(4-chlorophenyl)-2-pyrazinemethanol; N-(2,4-difluorophenyl)-N-benzyl-3-pyridinemethanamine and N-(2-chlorophenyl-$\alpha$-(4-fluorophenyl)-3-pyridinemethanamine; 1-(10,11-dihydro-5H-dibenzo[a,d]cyclohepten-5-yl)-1H-imidazole and 1-(9H-fluoren-9-yl)-1H-imidazole as disclosed in U.S. Patent No. 4,757,082; 3-bis(4-chlorophenyl)-3-methylpyridine and $\alpha,\alpha$-bis(4-

chlorophenyl)-3-pyridinemethanol; 5H-5-(4-cyanophenyl)-6,7-dihydropyrrolo[1,2-c]imidazole and 5H-5-(4-cyanophenyl)-6,7,8,9-tetrahydroimidazo[1,5-a]azepine; 5-[(1 H-imidazol-1-yl)phenylmethyl]-2-methyl-1 H-benzimidazole and 5-[(3-chlorophenyl)(1 H-imidazol-1-yl)-methyl]-1 H-benzimidazole; (Z)-α-(1,2,4-triazol-1-ylmethyl)stilbene-4,4'-dicarbonitrile, (Z)-4'-chloro-α-(1,2,4-triazol-1-ylmethyl)stilbene-4-carbonitrile, (Z)-α-(1,2,4-triazol-1-ylmethyl)-4'-(trifluoromethyl)stilbene-4-carbonitrile, (E)-ß-fluoro-α-(1,2,4-triazol-1-ylmethyl)stilbene-4,4'-dicarbonitrile, (Z)-4'-fluoro-α-(imidazol-1-ylmethyl)stilbene-4-carbonitrile, (Z)-2',4'-dichloro-α-(imidazol-1-ylmethyl)stilbene-4-carbonitrile, (Z)-4'-chloro-α-(imidazol-1-ylmethyl)-stilbene-4-carbonitrile, (Z)-α-(imidazol-1-ylmethyl)stilbene4,4'-dicarbonitrile,(Z)-α-(5-methylimidazol-1-ylmethyl)stilbene-4,4'-dicarbonitrile, and (Z)-2-[2-(4-cyanophenyl)-3-(1,2,4-triazol-1-yl)propenyl]pyridine-5-carbonitrile; (1R* ,2R*)-6-fluoro-2-(4-fluorophenyl)-1,2,3,4-tetrahydro-1-(1H-1,2,4-triazol-1-ylmethyl)-naphthalene, (1R* ,2R*)-6-fluoro-2-(4-fluorophenyl)-1,2,3,4-tetrahydro-1-(1H-imidazolylmethyl)-naphthalene, (1R* ,2R*)-and (1R* ,2S*)-2-(4-fluorophenyl)-1,2,3,4-tetrahydro-1-(1H-1,2,4-triazol-1-ylmethyl)-naphthalene-6-carbonitrile, (1R* ,2R*)-and (1R* ,2S*)-2-(4-fluorophenyl)-1,2,3,4-thetahydro-1-(1H-imidazolylmethyl)naphthalene-6-carbonitrile, (1R* ,2R*)-and (1R* ,2S*)-1,2,3,4-tetrahydro-1-(1H-1,2,4-triazol-1-ylmethyl)naphthalene-2,6-dicarbonitrile, (1R* ,2R*)-and (1R* ,2S*)-1,2,3,4-tetrahydro-1-(1H-imidazol-1-ylmethyl)naphthalene-2,6-dicarbonitrile, and(1R* ,2S*)-2-(4-fluorophenyl)-1,2,3,4-tetrahydro-1-(5-methyl-1H-imidazolylmethyl)naphthalene-6-carbonitrile; 8-chloro-5-(4-chlorophenyl)-5H-indeno[1,2-d] pyrimidine; 5-bis (4-chlorophenyl) methylpyrimidine; 10-(2-propynyl)-estr-4-ene-3,17-dione as disclosed in U.S. Patent No. 4,322,416; 6-[(4-chlorophenyl) (1H-1,2,4-triazol-1-yl) methyl]-1-methyl-1H-benzotriazole; 1-methylandrosta-1,4-dien-3,17-dione; 3-ethyl-3-(4-pyridyl)piperidine-2,6-dione; 4-hydroxyandrostene-3,17-dione; 4-(5,6,7,8-tetrahydroimidazo[1,5-a]pyridin-5yl)benzonitrile; 6-methyleneandrosta-1,4-diene-3,17-dione, 4-aminoadrostan-1,4,6-trien-3,17-dione and 4-aminoandrosta-4,6-diene-3,17-dione; 3-(1H-imidazol-1-ylmethyl)-2-methyl-1H-indole-1-propanoic acid as disclosed in U.S. Patent No. 4,273,782; 5-[3-chlorophenyl)(1H-imidazol-1-yl)methyl]-1H-benzimidazole; 10ß-thiiranylestr-4-ene-3,17-dione and 10ß-oxiranylestr-4-ene-3,17-dione; 3-ethyl-3-(4-pyridyl)piperidine-2,6-dione; 3-(4-aminophenyl)-3-ethyl-pyrrolidine-2,5-dione; 1-(7-carboxyheptyl)-imidazole; 1,1-dimethyl-8-(1H-1,2,4-triazol-1-ylmethyl)-2 (1H)-naphtho [2,1-b]furanone (1a); ± 5- (p-cyanophenyl)-5, 6, 7, 8-tetrahydroimidazo [1, 5-a] pyridine hydrochloride; 1,4,6-androstatriene-3,17-dione; and bis-(p-cyanophenyl)-imidazo-1-yl-methane hemisuccinate and pharmaceutically acceptable deritives, acid addition salts thereof.

6. The aromatase inhibitor of claim 5 wherein said aromatase inhibitor is ± 5- (p-cyanophenyl)-5, 6, 7, 8-tetrahydroimidazo [1, 5-α] pyridine hydrochloride.

7. The aromatase inhibitor of claim 5 wherein said aromatase inhibitor is 1,4,6-androstatriene-3,17-dione.

8. The method of claim 1 wherein the poultry embryos are treated with the aromatose inhibitor on about day 1 to about day 9 of incubation.

9. The method of claim 1 wherein the poultry embryos are treated with one or more aromatase inhibitors.

10. The method of claim 1 wherein poultry embryos are treated one or more times with an aromatase inhibitor.

11. Phenotypically male poultry comprising genotypic females treated with one or more aromatase inhibitor which converts the females to a male phenotype.

12. Genotypically female poultry having a male phenotype resulting from treating the developing embryo with one or more aromatase inhibitor.

13. A composition for converting genotypic female poultry into phenotypic male poultry comprising administering to a healthy poultry embryo a phenotypic converting amount of a substance which prevents the conversion of androgens to estrogens and results in the conversion of genotypic female poultry into phenotypic male poultry.

14. The composition for converting genotypic female poultry into phenotypic males of claim 13 wherein the phenotypic converting substance is one or more aromatase inhibitors in a physiologically acceptable carrier.

**15.** The aromatase inhibitor of claim 14 wherein the said aromatase inhibitor is a steroid substrate analogue or a non-steroid analogue which binds and inactivates the enzyme or a compound that inhibits the cytochrome P450 component of the enzymatic activity.

**16.** The aromatase inhibitor of claim 14 wherein the said aromatase inhibitor is selected from the group consisting of: 6-[(1H-imidazol-1-yl)phenylmethyl]-1 methyl-1H-benzotriazole, 6-[(4-chlorophenyl)(1H-1,2,4-triazol-1-yl)methyl]-1-methyl-1H-benzotriazole as described in Publication No. 293,978; 2,2-[5-(1H-1,2,4,-triazol-1-ylmethyl)-1,3-phenylene]di(2-methylpropiononitrile), 2,2-[5-(imidazol-1-ylmethyl)-1,3-phenylene]-di(2-methylpropiononitrile), 2-[3-(1-hydroxy-1-methylethyl)-5-(1H-1,2,4-triazol-1-ylmethylphenyl]-2-methylpropiononitrile, and 2,2-[5-dideuterio(1H-1,2,4-triazol-1-yl)methyl-1,3-phenylene]di(2trideuteriomethyl-3,3,3-(trideuteriopropiononitrile), and 2,2-[5-dideuterio(1H-1,2,4-triazol-1-ylmethyl-1,3-phenylene)di(2-methylpropiononitrile); 1,1-dimethyl-8-(1H-1,2,4-triazol-1-ylmethyl)-2(1H)-naphtho[2,1-b] furanone, 1,2-dihydro-1,1-dimethyl-2-oxo-8-(1H-1,2,4-triazol-1-ylmethyl)naphtho-[2,1-b]-furan-7-carbonitrile, 1,2-dihydro-1,1-dimethyl-2-oxo-8-(1H-1,2,4-triazol-1-ylmethyl)-naphtho[2,1-b]furan-7-carboxamide, and 1,2-dihydro-1,1-dimethyl-2-oxo-8[di(1H-1,2,4-triazol-1 yl)methyl]naphtho[2,1-b]furan-7-carbonitrile; 2-(4-chlorobenzyl)-2-fluoro-1,3-di(1,2,4-triazol-1-yl)propane, 2-fluoro-2-(2-fluoro-4-chlorobenzyl)-1,3-di(1,2,4-triazol-1-yl)propane, 2-fluoro-2-(2-fluoro-4-trifluoromethylbenzyl)-1,3-di(1,2,4-triazol-1-yl)-propane, 3-(4-chlorophenyl)-1-(1,2,4-triazol-1-yl)2-(1,2,4-triazol-1-ylmethyl)butan-2-ol, 2-(4-chloro-α-fluorobenzyl)-1,3-di(1,2,4-triazol-1-yl)propan-2-ol, 2-(4-chlorobenzyl)-1,3-bis(1,2,4-triazol-1-yl)propane, 4-[2-(4-chlorophenyl)-1,3-di(1,2,4-triazol-1-ylmethyl)ethoxymethyl]-benzonitrile,1-(4-fluorobenzyl)-2-(2-fluoro-4-trifluoromethylphenyl)-1,3-di(1,2,4-triazol-1-yl)propan-2-ol, 2-(4-chlorophenyl)-1-(4-fluorophenoxy)-1,3-di(1,2,4-triazol-1-yl)propan-2-ol, 1-(4-cyanobenzyl)-2-(2,4-difluorophenyl)-1,3-di(1,2,4-triazol-1-yl)-propan-2-ol and 2-(4-chlorophenyl)-1-phenyl-1,3-di(1,2,4-triazol-1-yl)-propan-2-ol; 5-bis(4-chlorophenyl)methylpyrimidine; α,α-bis(4-chlorophenyl)-2-pyrazinemethanol; N-(2,4-difluorophenyl)-N-benzyl-3-pyridinemethanamine and N-(2-chlorophenyl-α-(4-fluorophenyl)-3-pyridinemethanamine; 1-(10,11-dihydro-5H-dibenzo[a,d]cyclohepten-5-yl)-1H-imidazole and 1-(9H-fluoren-9-yl)-1H-imidazole as disclosed in U.S. Patent No. 4,757,082; 3-bis(4-chlorophenyl)-3-methylpyridine and α,α-bis(4-chlorophenyl)-3-pyridinemethanol; 5H-5-(4-cyanophenyl)-6,7-dihydropyrrolo[1,2-c]imidazole and 5H-5-(4-cyanophenyl)-6,7,8,9-tetrahydroimidazo[1,5-a]azepine; 5-[(1 H-imidazol-1-yl)phenylmethyl]-2-methyl-1 H-benzimidazole and 5-[(3-chlorophenyl)(1 H-imidazol-1-yl)-methyl]-1 H-benzimidazole; (Z)-α-(1,2,4-triazol-1-ylmethyl)stilbene-4,4'-dicarbonitrile, (Z)-4'-chloro-α-(1,2,4-triazol-1-ylmethyl)stilbene-4-carbonitrile, (Z)-α-(1,2,4-triazol-1-ylmethyl)-4'-(trifluoromethyl)stilbene-4-carbonitrile, (E)-β-fluoro-α-(1,2,4-triazol-1-ylmethyl)stilbene-4,4'-dicarbonitrile, (Z)-4'-fluoro-α-(imidazol-1-ylmethyl)stilbene-4-carbonitrile, (Z)-2',4'-dichloro-α-(imidazol-1-ylmethyl)stilbene-4-carbonitrile, (Z)-4'-chloro-α-(imidazol-1-ylmethyl)-stilbene-4-carbonitrile, (Z)-α-(imidazol-1-ylmethyl)stilbene4,4'-dicarbonitrile,(Z)-α-(5-methylimidazol-1-ylmethyl)stilbene-4,4'-dicarbonitrile, and (Z)-2-[2-[4-cyanophenyl)-3-(1,2,4-triazol-1-yl)propenyl]pyridine-5-carbonitrile; (1R* 2R*)-6-fluoro-2-(4-fluorophenyl)-1,2,3,4-tetrahydro-1-(1H-1,2,4-triazol-1-ylmethyl)-naphthalene, (1R* ,2R*)-6-fluoro-2-(4-fluorophenyl)-1,2,3,4-tetrahydro-1-(1H-imidazolylmethyl)-naphthalene, (1R* ,2R*)-and (1R* ,2S*)-2-(4-fluorophenyl)-1,2,3,4-tetrahydro-1-(1H-1,2,4-triazol-1-ylmethyl)-naphthalene-6-carbonitrile, (1R* ,2R*)-and (1R* ,2S*)-2-(4-fluorophenyl)-1,2,3,4-thetahydro-1-(1H-imidazolylmethyl)naphthalene-6-carbonitrile, (1R* ,2R*)-and (1R* ,2S*)-1,2,3,4-tetrahydro-1-(1H-1,2,4-triazol-1-ylmethyl)naphthalene-2,6-dicarbonitrile, (1R* ,2R*)-and (1R* ,2S*)-1,2,3,4-tetrahydro-1-(1H-imidazol-1-ylmethyl)naphthalene-2,6-dicarbonitrile, and(1R* ,2S*)-2-(4-fluorophenyl)-1,2,3,4-tetrahydro-1-(5-methyl-1H-imidazolylmethyl)naphthalene-6-carbonitrile; 8-chloro-5-(4-chlorophenyl)-5H-indeno[1,2-d] pyrimidine; 5-bis (4-chlorophenyl) methylpyrimidine; 10-(2-propynyl)-estr-4-ene-3,17-dione as disclosed in U.S. Patent No. 4,322,416; 6-[(4-chlorophenyl) (1H-1,2,4-triazol-1-yl) methyl]-1-methyl-1H-benzotriazole; 1-methylandrosta-1,4-dien-3,17-dione; 3-ethyl-3-(4-pyridyl)piperidine-2,6-dione; 4-hydroxyandrostene-3,17-dione; 4-(5,6,7,8-tetrahydroimidazo[1,5-a]pyridin-5yl)benzonitrile; 6-methyleneandrosta-1,4-diene-3,17-dione, 4-aminoadrostan-1,4,6-trien-3,17-dione and 4-aminoandrosta-4,6-diene-3,17-dione; 3-(1H-imidazol-1-ylmethyl)-2-methyl-1H-indole-1-propanoic acid as disclosed in U.S. Patent No. 4,273,782; 5-[3-chlorophenyl)(1H-imidazol-1-yl)methyl]-1H-benzimidazole; 10β-thiiranylestr-4-ene-3,17-dione and 10β-oxiranylestr-4-ene-3,17-dione; 3-ethyl-3-(4-pyridyl)piperidine-2,6-dione; 3-(4-aminophenyl)-3-ethyl-pyrrolidine-2,5-dione; 1-(7-carboxyheptyl)-imidazole; 1,1-dimethyl-8-(1H-1,2,4-triazol-1-ylmethyl)-2 (1H)-naphtho [2,1-b]furanone (1a); ± 5- (p-cyanophenyl)-5, 6, 7, 8-tetrahydroimidazo [1, 5-a] pyridine hydrochloride; 1,4,6-androstatriene-3,17-dione; and bis-(p-cyanophenyl)-imidazo-1-yl-methane hemisuccinate and pharmaceutically acceptable deritives, acid addition salts thereof.

**17.** The aromatose inhibitor of claim 16 wherein said aromatase inhibitor is ± 5- (p-cyanophenyl)-5, 6, 7, 8-tetrahydroimidazo [1, 5-α] pyridine hydrochloride.

**18.** The aromatase inhibitor of claim 16 wherein said aromatase inhibitor is 1,4,6-androstatriene-3,17-dione.

**19.** A method for increasing weight gain and/or an improved lean to fat ratio in genotypic female poultry comprising treatment of geontypic female poultry embryos with one or more aromatase inhibitors.

**20.** The aromatase inhibitor of claim 19 wherein the said aromatase inhibitor is selected from the group consisting of: 6-[(1H-imidazol-1-yl)phenylmethyl]-1 methyl-1H-benzotriazole, 6-[(4-chlorophenyl)(1H-1,2,4-triazol-1-yl)methyl]-1-methyl-1H-benzotriazole as described in Publication No. 293,978; 2,2-[5-(1H-1,2,4,-triazol-1-ylmethyl)-1,3-phenylene]di(2-methylpropiononitrile), 2,2-[5-(imidazol-1-ylmethyl)-1,3-phenylene]-di(2-methylpropiononitrile), 2-[3-(1-hydroxy-1-methylethyl)-5-(1H-1,2,4-triazol-1-ylmethylphenyl]-2-methylpropiononitrile, and 2,2-[5-dideuterio(1H-1,2,4-triazol-1-yl)methyl-1,3-phenylene]di(2trideuteriomethyl-3,3,3-(trideuteriopropiononitrile), and 2,2-[5-dideuterio(1H-1,2,4-triazol-1-ylmethyl-1,3-phenylene)di(2-methylpropiononitrile); 1,1-dimethyl-8-(1H-1,2,4-triazol-1-ylmethyl-2-(1H)-naphtho[2,1-b] furanone, 1,2-dihydro-1,1-dimethyl-2-oxo-8-(1H-1,2,4-triazol-1-ylmethyl)naphtho-[2,1-b]-furan-7-carbonitrile, 1,2-dihydro-1,1-dimethyl-2-oxo-8-(1H-1,2,4-triazol-1-ylmethyl)-naphtho[2,1-b]furan-7-carboxamide, and 1,2-dihydro-1,1-dimethyl-2-oxo-8-[di(1H-1,2,4-triazol-1 yl)methyl]naphtho[2,1-b]furan-7-carbonitrile; 2-(4-chlorobenzyl)-2-fluoro-1,3-di(1,2,4-triazol-1-yl)propane, 2-fluoro-2-(2-fluoro-4-chlorobenzyl)-1,3-di(1,2,4-triazol-1-yl)propane, 2-fluoro-2-(2-fluoro-4-trifluoromethylbenzyl)-1,3-di(1,2,4-triazol-1-yl)propane, 3-(4-chlorophenyl)-1-(1,2,4-triazol-1-yl)-2-(1,2,4-triazol-1-ylmethyl)butan-2-ol, 2-(4-chloro-α-fluorobenzyl)-1,3-di(1,2,4-triazol-1-yl)propan-2-ol, 2-(4-chlorobenzyl)-1,3-bis(1,2,4-triazol-1-yl)-propane, 4-[2-(4-chlorophenyl)-1,3-di(1,2,4-triazol-1-ylmethyl)ethoxymethyl]-benzonitrile, 1-(4-fluorobenzyl)2-(2-fluoro-4-trifluoromethylphenyl)-1,3-di(1,2,4-triazol-1-yl)propan-2-ol, 2-(4-chlorophenyl)-1-(4-fluorophenoxy)-1,3-di(1,2,4-triazol-1-yl)propan-2-ol, 1-(4-cyanobenzyl)-2-(2,4-difluorophenyl)-1,3-di(1,2,4-triazol-1-yl)-propan-2-ol and 2-(4-chlorophenyl)-1-phenyl-1,3-di(1,2,4-triazol-1-yl)-propan-2-ol; 5-bis(4-chlorophenyl)methylpyrimidine; α,α-bis(4-chlorophenyl)-2-pyrazinemethanol; N-(2,4-difluorophenyl)-N-benzyl-3-pyridinemethanamine and N-(2-chlorophenyl-α-(4 fluorophenyl)-3-pyridinemethanamine; 1-(10,11-dihydro-5H-dibenzo[a,d]cyclohepten-5-yl)-1H-imidazole and 1-(9H-fluoren-9-yl)-1H-imidazole as disclosed in U.S. Patent No. 4,757,082; 3-bis(4-chlorophenyl)-3-methylpyridine and α,α-bis(4-chlorophenyl)-3-pyridinemethanol; 5H-5-(4-cyanophenyl)-6,7-dihydropyrrolo[1,2-c]imidazole and 5H-5-(4-cyanophenyl)-6,7,8,9-tetrahydroimidazo[1,5-a]azepine; 5-[(1H-imidazol-1-yl)phenylmethyl]-2-methyl-1 H-benzimidazole and 5-[(3-chlorophenyl)(1 H-imidazol-1-yl)-methyl]-1 H-benzimidazole; (Z)-α-(1,2,4-triazol-1-ylmethyl)stilbene-4,4'-dicarbonitrile, (Z)-4'-chloro-α-(1,2,4-triazol-1-ylmethyl)stilbene-4-carbonitrile, (Z)-α-(1,2,4-triazol-1-ylmethyl)-4'-(trifluoromethyl)stilbene-4-carbonitrile, (E)-β-fluoro-α-(1,2,4-triazol-1-ylmethyl)stilbene-4,4'-dicarbonitrile, (Z)-4'-fluoro-α-(imidazol-1-ylmethyl)stilbene-4-carbonitrile, (Z)-2',4'-dichloro-α-(imidazol-1-ylmethyl)stilbene-4-carbonitrile, (Z)-4'-chloro-α-(imidazol-1-ylmethyl)-stilbene-4-carbonitrile, (Z)-α-(imidazol-1-ylmethyl)stilbene4,4'-dicarbonitrile,(Z)-α-(5-methylimidazol-1-ylmethyl)stilbene-4,4'-dicarbonitrile, and (Z)-2-[2-(4-cyanophenyl)-3-(1,2,4-triazol-1-yl)propenyl]pyridine-5-carbonitrile; (1R* ,2R*)-6-fluoro-2-(4-fluorophenyl)-1,2,3,4-tetrahydro-1-(1H-1,2,4-triazol-1-ylmethyl)-naphthalene, (1R* ,2R*)-6-fluoro-2-(4-fluorophenyl)-1,2,3,4-tetrahydro-1-(1H-imidazolylmethyl)-naphthalene, (1R* ,2R*)-and (1R* ,2S*)-2-(4-fluorophenyl)-1,2,3,4-tetrahydro-1-(1H-1,2,4-triazol-1-ylmethyl)-naphthalene-6-carbonitrile, (1R* ,2R*)-and (1R* ,2S*)-2-(4-fluorophenyl)-1,2,3,4-thetahydro-1-(1H-imidazolylmethyl)naphthalene-6-carbonitrile, (1R* ,2R*)-and (1R* ,2S*)-1,2,3,4-tetrahydro-1-(1H-1,2,4-triazol-1-ylmethyl)naphthalene-2,6-dicarbonitrile, (1R* ,2R*)-and (1R* ,2S*)-1,2,3,4-tetrahydro-1-(1H-imidazol-1-ylmethyl)naphthalene-2,6-dicarbonitrile, and(1R* ,2S*)-2-(4-fluorophenyl)-1,2,3,4-tetrahydro-1-(5-methyl-1H-imidazolylmethyl)naphthalene-6-carbonitrile; 8-chloro-5-(4-chlorophenyl)-5H-indeno[1,2-d]pyrimidine; 5-bis (4-chlorophenyl) methylpyrimidine; 10-(2-propynyl)-estr-4-ene-3,17-dione as disclosed in U.S. Patent No. 4,322,416; 6-[(4-chlorophenyl) (1H-1,2,4-triazol-1-yl) methyl]-1-methyl-1H-benzotriazole; 1-methylandrosta-1,4-dien-3,17-dione; 3-ethyl-3-(4-pyridyl)piperidine-2,6-dione; 4-hydroxyandrostene-3,17-dione; 4-(5,6,7,8-tetrahydroimidazo[1,5-a]pyridin-5yl)benzonitrile; 6-methyleneandrosta-1,4-diene-3,17-dione, 4-aminoadrostan-1,4,6-trien-3,17-dione and 4-aminoandrosta-4,6-diene-3,17-dione; 3-(1H-imidazol-1-ylmethyl)-2-methyl-1H-indole-1-propanoic acid as disclosed in U.S. Patent No. 4,273,782; 5-[3-chlorophenyl)(1H-imidazol-1-yl)methyl]-1H-benzimidazole; 10β-thiiranylestr-4-ene-3,17-dione and 10β-oxiranylestr-4-ene-3,17-dione; 3-ethyl-3-(4-pyridyl)piperidine-2,6-dione; 3-(4-aminophenyl)-3-ethyl-pyrrolidine-2,5-dione; 1-(7-carboxyheptyl)-imidazole; 1,1-dimethyl-8-(1H-1,2,4-triazol-1-ylmethyl)-2 (1H)-naphtho [2,1-b]furanone (1a); ± 5- (p-cyanophenyl)-5, 6, 7, 8-tetrahydroimidazo [1, 5-a] pyridine hydrochloride; 1,4,6-androstatriene-3, 17-dione; and bis-(p-cyanophenyl)-imidazo-1-yl-methane hemisuccinate and pharmaceutically acceptable deritives, acid addition salts thereof.

21. The aromatose inhibitor of claim 20 wherein said aromatase inhibitor is ± 5- (p-cyanophenyl)-5, 6, 7, 8-tetrahydroimidazo [1, 5-α] pyridine hydrochloride.

22. The aromatase inhibitor of claim 20 wherein said aromatase inhibitor is 1,4,6-androstatriene-3,17-dione.

pUGD0603   pOV230

female

male

1976

1977

1978

1981